# EUROPÄISCHE PATENTSCHRIFT

(11) **EP 0 342 532 B1**
(45) Veröffentlichungstag und Bekanntmachung des Hinweises auf die Patenterteilung: **07.01.1993**
(21) Anmeldenummer: 89108539.1
(22) Anmeldetag: 12.05.1989
(51) Int. Cl.: C07C 205/13, C07C 201/06

(54) **Verfahren zur Herstellung von 4-Chlor-2-methyl-5-nitro-phenol**
Process for the preparation of 4-chloro-2-methyl-5-nitro-phenol
Procédé pour la préparation du 4-chloro-2-méthyl-5-nitro-phénol

(30) Priorität: 18.05.1988 DE 3816839
(43) Veröffentlichungstag der Anmeldung: 23.11.1989
(73) Patentinhaber: Wella Aktiengesellschaft, 64295 Darmstadt (DE)
(72) Erfinder: Clausen, Thomas, Dr., D-6146 Alsbach (DE); Rose, David, Dr., D-4010 Hilden (DE)

(56) Entgegenhaltungen:
- DE-B- 1 260 468
- FR-A- 1 504 228
- FR-A- 2 106 907
- GB-A- 984 305
- US-A- 3 636 037
- T.W. GREENE:"PROCTECTIVE GROUPS IN ORGANIC SYNTHESIS", 1981, John Wiley & Sons, New York, US
- PATENT ABSTRACTS OF JAPAN, Band 7, Nr. 175 (C-179)(1320), 3. August 1983; & JP-A-58083652

## Beschreibung

Für die Verwendung in Oxidationshaarfärbemitteln haben Derivate des m-Aminophenols eine große Bedeutung als Kupplungskomponenten für den Rotbereich gewonnen. In jüngerer Zeit wurden neue m-Aminophenolderivate, die sich durch hohe Lichtechtheit und gute toxikologische Eigenschaften auszeichnen sollen, hergestellt. Derartige m-Aminophenolderivate sind beispielsweise in der DE-OS 3 524 329 beschrieben. Die entscheidende Vorstufe für die Herstellung aller dort genannten Verbindungen ist das 4-Chlor-2-methyl-5-nitro-phenol (I) 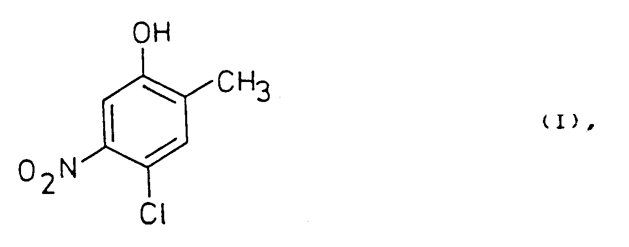
dessen Herstellung aus der GB-PS 1 100 219 bekannt ist.

Das dort beschriebene Verfahren geht vom 4-Chloro-2-methyl-anilin aus, welches zunächst nitriert wird und anschließend durch Diazotierung und Verkochung in das Nitrophenol (I) übergeführt wird. In neuerer Zeit wurde jedoch bekannt, daß das 4-Chlor-2-methyl-anilin Blasenkrebs verursachen kann. Deshalb kann das vorstehend beschriebene Herstellungsverfahren in technischem Maßstab nicht mehr durchgeführt werden.

Die Nitrierung des technisch verfügbaren und sehr preiswerten 4-Chlor-2-methyl-phenols ist in der Literatur Zincke, Liebigs Ann. Chem. Seite 417, 222 beschrieben. Jene Umsetzung ergibt jedoch das 4-Chlor-2-methyl-6-nitro-phenol, welchen ein Isomeres zu der Verbindung (I) darstellt.

Ein weiteres Verfahren zur Herstellung des 4-Chlor-2-methyl-5-nitro-phenols ist in der FR-PS 2 106 907 beschrieben. Dort wird der Bis-Kohlensäureester des 4-Chlor-2-methyl-phenols zunächst nitriert und anschließend durch alkalische Hydrolyse gespalten. Nachteile jenes Verfahrens sind die erforderliche Darstellung des Bis-Kohlensäureesters, welche mit Phosgen oder dessen Derivaten erfolgt und damit zu Sicherheitsproblemen führt, sowie die geringe Ausbeute bei der Nitrierung. Die durch die drastischen Reaktionsbedingungen hervorgerufene Bildung isomerer Produkte erfordert einen zusätzlichen Reinigungsschritt, nach dessen Durchführung die Ausbeute nur 57 Prozent der Theorie beträgt.

Es bestand daher die Aufgabe, ein Herstellungsverfahren für 4-Chlor-2-methyl-5-nitro-phenol (I) zur Verfügung zu stellen, welches, ausgehend von technisch verfügbaren und preiswerten Ausgangsstoffen, die isomerenreine Herstellung von (I) in sehr guter Ausbeute ermöglicht.

Es wurde nun gefunden, daß sich die Verbindung (I) aus dem Ausgangsstoff 4-Chlor-2-methyl-phenol problemlos und in sehr guter Ausbeute herstellen läßt, wenn man zunächst die Hydroxygruppe mit einem Sulfonylrest schützt, das so entstandene Zwischenprodukt sodann nitriert und anschließend den Sulfonylrest wieder abspaltet.

Aus der Veröffentlichung L.G. Groves et. al., J. Chem. Soc., 512 bis 524 (1929) ist bekannt, 2,4-Dichlorphenol zu 2,4-Dichlor-5-nitrophenol unter Verwendung einer Sulfonylschutzgruppe umzusetzen, wobei jedoch Nebenprodukte entstehen.

Gegenstand der Erfindung ist daher ein Verfahren zur Herstellung von 4-Chlor-2-methyl-5-nitro-phenol (I) 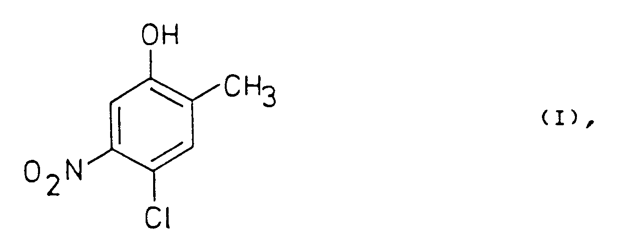
dadurch gekennzeichnet, daß ein 4-Chlor-2-methyl-phenyl-sulfonat der Formel (II) 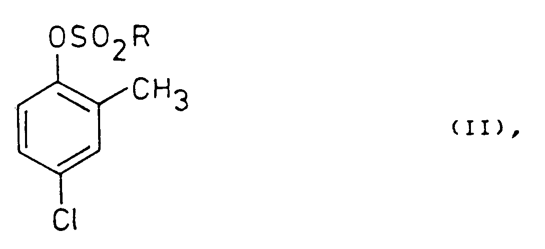
worin R einen der Reste Methyl, Ethyl, Trifluormethyl, Phenyl oder Tolyl bedeutet, zunächst in 5-Stellung nitriert wird und sodann das entstandene 4-Chlor-2-methyl-5-nitro-phenyl-sulfonat durch saure oder alkalische Abspaltung des Sulfonylrestes in die Verbindung der Formel (I) überführt wird.

Der besondere Wert des Verfahrens liegt darin, daß mit Hilfe des zwischenzeitlich eingeführten Sulfonylrestes die eintretende Nitrogruppe in die 5-Stellung dirigiert wird, wobei mit 4-Chlor-2-methyl-phenol ein sehr preiswertes Ausgangsmaterial verwendet werden kann. Die Nitrierung erfolgt unter schonenden Bedingungen, wobei der Sulfonylrest während der Reaktion nicht angegriffen wird.

Die Verbindungen der Formel (II) sind entweder wie im Falle R = Phenyl oder Tolyl bekannt (zum Beispiel GB-PS 984 305; A.R. Parikh et al., J. Inst. Chemists (India), Vol. XLV, May 1973 S. 91) oder lassen sich in analoger Weise gemäß den in den nachfolgenden Beispielen für R = Methyl und Phenyl beschriebenen Verfahren herstellen.

Obwohl das erfindungsgemäße Verfahren auch mit weiteren in der Literatur, beispielsweise bei T. W. Greene, Protective Groups in Organic Chemistry, Wiley Interscience, Neu York (1981), beschriebenen Sulfonatschutzgruppen durchgeführt werden kann, seien das Methan-, Ethan-, Trifluormethan-, Benzol- oder Toluolsulfonat, als besonders geeignet hervorgehoben.

Von besonderer Bedeutung hat sich jenes erfindungsgemäße Verfahren erwiesen, worin die Verbindung der Formel (II) das 4-Chlor-2-methyl-phenyl-methansulfonat ist. Das 4-Chlor-2-methyl-phenyl-methansulfonat wird durch Umsetzung von 4-chlor-2-methyl-phenol mit Methansulfonsäurechlorid in Gegenwart von Pyridin hergestellt, wobei das Produkt durch Zugabe von Eis und nachfolgende Extraktion mit Ether isoliert wird. Anschließend wird das 4-Chlor-2-methyl-phenyl-methansulfonats mit einem Gemisch aus Schwefelsäure und Salpetersäure bei etwa 0 Grad Celsius nitriert. Schließlich erfolgt die Abspaltung des Methansulfonylrestes entweder durch saure Esterspaltung, beispielsweise durch Erhitzen des 4-Chlor-2-methyl-5-nitro-phenyl-methansulfonats in konzentrierter Salzsäure bei einer Temperatur von etwa 80 Grad Celsius oder durch alkalische Esterspaltung, beispielsweise durch Umsetzung des 4-Chlor-2-methyl-5-nitro-phenyl-methansulfonats mit Natriummethylat oder methanolischer Kaliumhydroxidlösung bei Raumtemperatur, wobei das gewünschte Produkt 4-chlor-2-methyl-5-nitro-phenol (I) rein und in guter Ausbeute anfällt.

Als besonders geeignet für die Herstellung von 4-Chlor-2-methyl-5-nitro-phenol (I) in technischem Maßstab hat sich jene Ausführungsform des erfindungsgemäßen Verfahrens erwiesen, worin die Verbindung der Formel (II) das 4-Chlor-2-methyl-phenyl-benzolsulfonat ist. Die Herstellung des 4-Chlor-phenyl-2-methyl-benzolsulfonats durch Umsetzung von 4-Chlor-2-methyl-phenol mit Benzolsulfonsäurechlorid ist beispielsweise aus der GB-PS 984 305 und der Veröffentlichung Le Van Thoi et al., Ann. Fac. Sci. Univ. Saigon 1962, Seiten 73-88; CA 62, 2730b bekannt. Während im ersten Fall Pyridin als Base und Lösungsmittel diente, wurde im zweiten Fall als Base Alkalihydroxid und als Lösungsmittel Aceton verwendet. In beiden Fällen ist ein weiterer Arbeitsschritt der Isolierung des Produktes aus dem jeweiligen Lösungsmittel notwendig.

Es wurde gefunden, daß man das 4-Chlor-2-methyl-phenyl-benzolsulfonat direkt in seiner kristallinen Form erhält, wenn eine wäßrige Natriumhydroxidlösung zu einem auf etwa 60 bis 70 Grad Celsius erwärmten Gemisch aus 4-Chlor-2-methyl-phenol und Benzolsulfonsäure-chlorid getropft wird.

Die Nitrierung des 4-Chlor-2-methyl-phenyl-benzolsulfonats kann im erfindungsgemäßen Verfahren bereits bei etwa 15 Grad Celsius erfolgen, ohne daß Nebenprodukte entstehen. Vorzugsweise erfolgt die Nitrierung bei 15 bis 17 Grad Celsius. Sie führt zu dem 4-Chlor-2-methyl-5-nitrophenyl-benzolsulfonat, welches sich leicht durch Umkristallisation reinigen läßt.

Die Abspaltung des Benzolsulfonylrestes wird mit wäßriger Natriumhydroxidlösung bei Rückflußtemperatur durchgeführt, wobei das gewünschte Produkt 4-Chlor-2-methyl-5-nitrophenol (I) rein und in sehr guter Ausbeute anfällt.

Die nachfolgenden Beispiele sollen den Gegenstand der Erfindung näher erläutern.

### Beispiele zur Herstellung von 4-Chlor-2-methyl-5-nitro-phenol (I)

### Beispiel A: Herstellung von (I) unter Verwendung der Methansulfonylschutzgruppe

### Stufe 1: 4-Chlor-2-methyl-phenyl-methansulfonat

90,0 g (0,63 mol) 4-Chlor-2-methyl-phenol werden mit 81 ml (1,0 ml) Pyridin vermischt. Unter Rühren läßt man 57 ml (0,75 mol) Methansulfonsäurechlorid so zutropfen, daß die Temperatur nicht über 80 Grad Celsius steigt. Nach beendetem Zutropfen wird der Ansatz 2 Stunden lang auf 80 Grad Celsius erwärmt. Anschließend wird der Ansatz auf Eis gegossen, das Gemisch mit Ether extrahiert und die vereinigten Etherphasen werden mit 2 n Salzsäure und Wasser neutral gewaschen. Nach dem Trocknen der Etherphase verbleibt der Sulfonsäureester als fast farblose wachsartige Masse, die ohne Reinigung in Stufe 2 eingesetzt kann. Die Ausbeute beträgt 129,1 g (94 Prozent der Theorie). Eine Reinigung durch Absaugen der wachsartigen Masse unter Nachwaschen mit Methanol führt zu farblosen kristallinen Nadeln, die nach dem Trocknen bei 34 - 35 Grad Celsius schmelzen.

### Stufe 2: 4-Chlor-2-methyl-5-nitro-phenyl-methansulfonat

20,0 g (91 mmol) des Sulfonsäureesters aus der Stufe 1 läßt man unter Kühlen und Rühren zu 100 ml (1877 mmol) konzentrierter Schwefelsäure zutropfen, anschließend werden bei -5 bis 0 Grad Celsius 6 ml (133 mmol) konzentrierte Salpetersäure tropfenweise zugesetzt. Der Ansatz wird 130 Minuten lang bei 0 Grad Celsius gerührt und anschließend auf Eis gegossen. Sodann werden die ausgefallenen Kristalle mit Wasser nachgewaschen und durch Absaugen isoliert. Nach dem Trocknen erhält man 22,9 g (95 Prozent der Theorie) der Nitroverbindung mit einem Schmelzpunkt von 97 bis 99 Grad Celsius. Die Verwendung des kristallinen Esters aus der Stufe 1 als Ausgangsmaterial liefert ein isomerenfreies reines 4-Chlor-2-methyl-5-nitro-phenyl-methansulfonat mit einem Schmelzpunkt von 99 Grad Celsius.

### Stufe 3: 4-Chlor-2-methyl-5-nitro-phenol (I)

### alkalische Esterspaltung

a) 5,0 g (19 mmol) des Esters aus der Stufe 2 werden bei Raumtemperatur in einer Lösung aus 4,2 g (78 mmol) Natriummethylat in 100 ml Methanol gelöst. Nach einstündigem Rühren bei Raumtemperatur wird der Hauptteil des Lösungsmittels im Vakuum verdampft. Nach der Neutralisation mit 2 n Salzsäure erhält man 3,5 g (99 Prozent der Theorie) des reinen Phenols (I) mit einem Schmelzpunkt von 134 Grad Celsius.
b) 13,25 g (50 mmol) des Esters aus der Stufe 2 werden in 50 ml Methanol aufgeschlämmt, wobei sich ein Teil des Esters löst. Bei Zimmertemperatur werden 20 ml einer 20 prozentigen methanolischen Kaliumhydroxidlösung zugesetzt. Dabei erwärmt sich die Mischung auf 36 Grad Celsius, färbt sich orange und gleichzeitig tritt ein Niederschlag auf. Nach 1 stündigem Rühren gießt man den Ansatz in 400 ml Wasser und säuert mit Essigsäure an. Das reine Phenol (I) fällt aus und wird abgesaugt.
   Ausbeute 7,7 g (83 Prozent der Theorie) saure Esterspaltung:
c) 4,3 g (16 mmol) des Esters aus der Stufe 2 werden über Nacht in 20 ml konzentrierter Salzsäure auf 80 Grad Celsius erwärmt. Nach dem Abkühlen kristallisiert das Phenol aus, das durch Absaugen und Nachwaschen mit Wasser isoliert wird. Nach dem Trocknen erhält man 2,2 g (73 Prozent der Theorie) des reinen Phenols (I).

### Beispiel B: Herstellung von (I) unter Verwendung der Benzolsulfonylschutzgruppe

### Stufe 1: 4-Chlor-2-methyl-phenyl-benzolsulfonat

Eine Mischung aus 114,1 g (0,8 mol) 4-Chlor-2-methyl-phenol und 141,3 g (0,8 mol) Benzolsulfonsäurechlorid wird auf 60 bis 70 Grad Celsius erwärmt. Man läßt eine Lösung von 32 g (0,8 mol) Natriumhydroxid in 1,1 l Wasser innerhalb eines Zeitraums von 30 Minuten zutropfen und rührt anschließend den Ansatz 2 Stunden lang bei 60 Grad Celsius. Danach wird die Mischung abgekühlt und der Niederschlag abgesaugt. Die Ausbeute beträgt 202,1 g (89,3 Prozent der Theorie). Das Produkt kann ohne weitere Reinigung in Stufe 2 eingesetzt werden.

### Stufe 2: 4-Chlor-2-methyl-5-nitro-phenyl-benzolsulfonat

113 g (0,4 mol) des Sulfonsäureesters aus der Stufe 1 werden bei Raumtemperatur unter kräftigem Rühren zu 450 ml konzentrierter Schwefelsäure gegeben. Anschließend werden bei 15 bis 17 Grad Celsius innerhalb eines Zeitraumes von 3 Stunden 41 g (0,42 mol) konzentrierte Salpetersäure zugetropft. Nach 2 stündigem Rühren bei 15 Grad Celsius wird der Ansatz auf Eis gegossen und die ausgefallenen Kristalle werden abgesaugt. Nach dem Waschem mit Wasser werden die Kristalle bei 50 Grad Celsius im Vakuum getrocknet. Die Ausbeute beträgt 122,3 g. Das Produkt wird aus Ethanol umkristallisiert. Die nach dem Umkristallisieren erhaltenen gelben Nadeln besitzen einen Schmelzpunkt von 99 bis 105 Grad Celsius. Die vorstehend beschriebene Reaktion kann auch mit der Hälfte der Menge der Schwefelsäure durchgeführt werden.

### Stufe 3: 4-Chlor-2-methyl-5-nitro-phenol

84,2 g (0,26 mol) des Esters aus der Stufe 2 werden in 1 l Wasser suspendiert und mit 30,8 g (0,77 mol) Natriumhydroxid-Plätzchen versetzt. Nach 2 stündigem Kochen unter Rückfluß wird der Ansatz abgekühlt und die Lösung wird mit 2 n Salzsäure auf einen pH-Wert von 2,1 gestellt. Der ausgefallene Niederschlag wird abgesaugt, mit Wasser nachgewaschen und im Vakuum bei 50 Grad Celsius getrocknet. Die Ausbeute beträgt 45,6 g (95,4 Prozent der Theorie).

## Patentansprüche

1. Verfahren zur Herstellung von 4-Chlor-2-methyl-5-nitro-phenol (I) 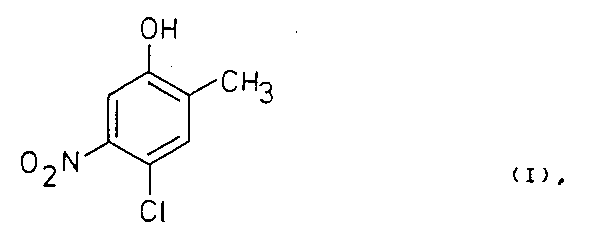 dadurch gekennzeichnet, daß ein 4-Chlor-2-methyl-phenyl-sulfonat der Formel (II) 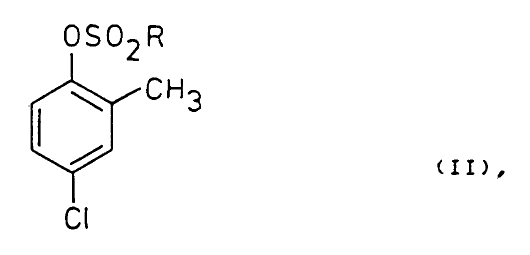 worin R einen der Reste Methyl, Ethyl, Trifluormethyl, Phenyl oder Tolyl bedeutet, zunächst in 5-Stellung nitriert wird und sodann das entstandene 4-Chlor-2-methyl-5-nitro-phenyl-sulfonat durch saure oder alkalische Abspaltung des Sulfonylrestes in die Verbindung der Formel (I) überführt wird.

2. Verfahren nach Anspruch 1, dadurch gekennzeichnet, daß als Verbindung der Formel (II) das 4-Chlor-2-methyl-phenyl-methansulfonat eingesetzt wird.

3. Verfahren nach Anspruch 2, dadurch gekennzeichnet, daß das 4-Chlor-2-methyl-phenyl-methansulfonat durch Umsetzung von 4-Chlor-2-methyl-phenol mit Methansulfonsäurechlorid in Gegenwart von Pyridin hergestellt wird.

4. Verfahren nach einem der Ansprüche 2 und 3, dadurch gekennzeichnet, daß die Abspaltung des Methansulfonylrestes mit konzentrierter Salzsäure erfolgt.

5. Verfahren nach einem der Ansprüche 2 bis 3 dadurch gekennzeichnet, daß die Abspaltung des Methansulfonylrestes mit Natriummethylat oder methanolischer Kaliumhydroxidlösung erfolgt.

6. Verfahren nach Anspruch 1, dadurch gekennzeichnet, daß als Verbindung der Formel (II) das 4-Chlor-2-methyl-phenyl-benzolsulfonat eingesetzt wird.

7. Verfahren nach Anspruch 6, dadurch gekennzeichnet, daß das 4-Chlor-2-methyl-phenyl-benzolsulfonat durch Umsetzung von 4-Chlor-2-methyl-phenol mit Benzolsulfonsäurechlorid in Gegenwart von wäßriger Natriumhydroxidlösung hergestellt wird.

8. Verfahren nach Anspruch 7, dadurch gekennzeichnet, daß die Umsetzung von 4-Chlor-2-methyl-phenol mit Benzolsulfonsäurechlorid bei 60 bis 70 Grad Celsius erfolgt.

9. Verfahren nach einem der Ansprüche 6 bis 8, dadurch gekennzeichnet, daß die Nitrierung bei 15 bis 17 Grad Celsius erfolgt.

10. Verfahren nach einem der Ansprüche 6 bis 9, dadurch gekennzeichnet, daß die Abspaltung des Benzolsulfonylrestes mit wäßriger Natriumhydroxidlösung erfolgt.

## Claims

1. Process for the the production of 4-chloro-2-methyl-5-nitro-phenol (I) 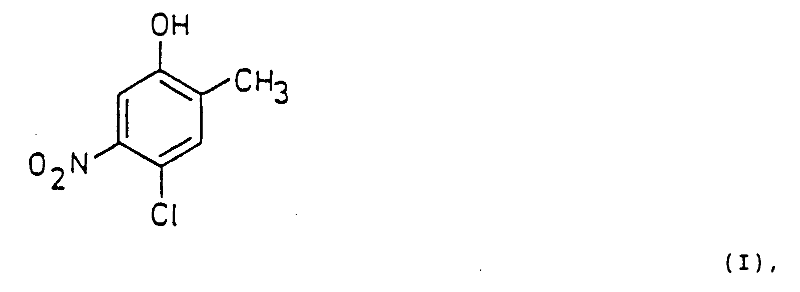 characterised in that a 4-chloro-2-methyl-phenyl-sulphonate of formula (II) 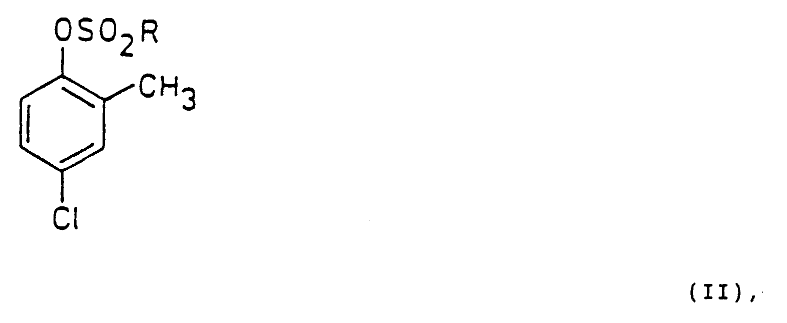 in which R signifies one of the residues methyl, ethyl, trifluoromethyl, phenyl or tolyl, is firstly nitrated in the 5-position and the resultant 4-chloro-2-methyl-5-nitro-phenylsulphonate is converted into the compound of formula (I) by acid or alkaline cleavage of the sulphonyl residue.

2. Process according to Claim 1, characterised in that 4-chloro-2-methyl-phenyl-methane-sulphonate is used as the compound according to formula (II).

3. Process according to Claim 2, characterised in that 4-chloro-2-methyl-phenyl-methane-sulphonate is produced by reacting 4-chloro-2-methyl-phenol with methane sulphonic acid chloride in the presence of pyridine.

4. Process as claimed in either of Claims 2 and 3, characterised in that the methane sulphonyl residue is cleaved with concentrated hydrochloric acid.

5. Process according to either of Claims 2 and 3, characterised in that the methane sulphonyl residue is cleaved with sodium methylate or methanolic potassium hydroxide solution.

6. Process according to Claim 1, characterised in that 4-chloro-2-methyl-phenyl-benzene-sulphonate is used as the compound according to formula (II).

7. Process according to Claim 6, characterised in that 4-chloro-2-methyl-phenyl-benzene-sulphonate is produced by reacting 4-chloro-2-methyl-phenol with benzene sulphonic acid chloride in the presence of an aqueous sodium hydroxide solution.

8. Process according to Claim 7, characterised in that the reaction between 4-chloro-2-methyl-phenol and benzene sulphonic acid chloride is carried out at 60 - 70 degrees Celsius.

9. Process according to any of Claims 6 to 8, characterised in that the nitration is carried out at between 15 and 17 degrees Celsius.

10. Process according to any of Claims 6 to 9, characterised in that the benzene sulphonyl residue is separated with aqueous sodium hydroxide solution.

## Revendications

1. Procédé de préparation de 4-chloro-2-methyl-5-nitro-phénol (I) 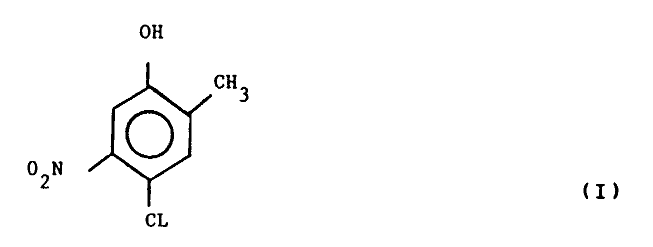 Caractérisé en ce qu'un 4-Chloro-2-méthyl-phényl-sulfonate de formule (II) : 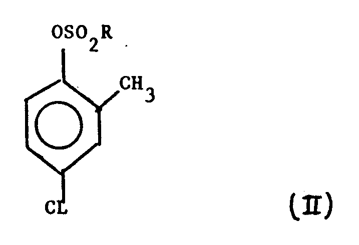 dans lequel R représente un reste méthyle, éthyle, trifluorométhyle, phényle ou tolyle, est nitré d'abord en position 5 et ensuite on transforme le 4-Chloro-2-methyl-5-nitro-phényle sulfonate formé par dissociation acide ou alcaline du reste sulfonyle dans la combinaison de la formule (I).

2. Procédé selon la revendication 1, caractérisé en ce qu'on utilise du 4-Chloro-2-méthyl-phényl-méthane-sulfonate comme composé de formule (II).

3. Procédé selon la revendication 2, caractérisé en ce que le 4-Chloro-2-méthyl-phényl-méthane sulfonate est obtenu en faisant réagir du 4-Chloro-2-méthyl-phénol avec du Chlorosulfonate de méthyle en présence de piridine.

4. Procédé selon l'une des revendiations 2 et 3, caractérisé en ce que la séparation du reste méthane sulfonyle s'effectue avec de l'acide chlorhydrique concentré.

5. Procédé selon l'une des revendications 2 à 3, caractérisé en ce que la séparation du reste méthane sulfonyle s'effectue avec du méthylate de sodium ou une solution méthanolique d'hydroxyde de potassium.

6. Procédé selon la revendaication 1, caractérisé en ce que l'on utilise le 4-Chloro-2-méthyl-phényl-benzène sulfonate, comme composé de formule (II).

7. Procédé selon la revendication 6, caractérisé en ce que le 4-Chloro-2-méthyl phényl benzene sulfonate est obtenu en faisant réagir du 4-chloro-2-méthyl phénol avec du Chlorosulfonate de benzène en présence d'une solution aqueuse d'hydroxyde de sodium.

8. Procédé selon la revendication 7, caractérisé en ce que la réaction du 4-Chloro-2-méthyl-phénol avec le chlorosulfonate de benzène s'effectue de 60 à 70 °C.

9. Procédé selon l'une des revendiations 6 à 8, caractérisé en ce que la nitration s'effectue de 15 à 17 °C.

10. Procédé selon l'une des revendications 6 à 9, caractérisé en ce que la séparation du reste benzène sulfonyle s'effectue avec une solution aqueuse d'hydroxyde de sodium.
